# FASCICULE DE BREVET EUROPEEN

(11) **EP 0 889 757 B1**
(45) Date de publication et mention de la délivrance du brevet: **12.06.2002**
(21) Numéro de dépôt: 97916509.9
(22) Date de dépôt: 27.03.1997
(51) Int. Cl.: B05B 11/00, A61M 15/00, A61L 2/16

(54) **DISPOSITIF ANTIBACTERIEN DE PULVERISATION D'UN PRODUIT LIQUIDE**
ANTIBAKTERIELLE SPRÜHVORRICHTUNG FÜR EINE FLÜSSIGKEIT
ANTIBACTERIAL DEVICE FOR SPRAYING A LIQUID

(30) Priorité: 29.03.1996 FR 9603943
(43) Date de publication de la demande: 13.01.1999
(73) Titulaire: REXAM SOFAB, 76450 Le Tréport (FR)
(72) Inventeur: HENNEMANN, Pascal, F-76260 Eu (FR); PROST, Frédéric, F-75008 Paris (FR); BOUGAMONT, Jean-Louis, F-76260 Eu (FR)
(74) Mandataire: Busnel, Jean-Benoît
(86) Numéro de dépôt international: FR9700550
(87) Numéro de publication internationale: WO9736690

(56) Documents cités:
- FR-A- 2 635 084
- GB-A- 898 320
- US-A- 4 830 284
- US-A- 5 181 658
- US-A- 5 186 368
- US-A- 5 360 145

## Description

La présente invention concerne un dispositif antibactérien de pulvérisation d'un produit liquide.

Le produit liquide est contenu dans un récipient et la pulvérisation est généralement effectuée par un dispositif traditionnel comprenant, notamment, un embout destiné à coiffer le tube gicleur d'une pompe montée sur ledit récipient au moyen d'une collerette.

Un tel dispositif est décrit, par exemple, dans le US-A 4 830 284 où ledit embout comportant un conduit interne prolongeant, d'une part, le tube gicleur jusqu'à une cavité d'extrémité pourvue d'un orifice d'éjection, dans laquelle est logé un clapet, et entouré coaxialement, d'autre part, d'une paroi externe cylindro-conique raccordée en partie basse à un épaulement d'appui pour la manoeuvre de la pompe.

Avec de tels dispositifs, la protection anti-bactérienne du produit n'est assurée que par les clapets de la pompe. Cette protection est complétée, le cas échéant, par l'utilisation d'une poche déformable renfermant le produit, et qui est soudée à la pompe, ou d'un récipient de volume variable pourvu d'un fond mobile.

Cependant, ces moyens de protection ne sont pas suffisamment efficaces pour maintenir le conditionnement stérile et pour éviter tout risque de contamination du produit.

Une autre technique de protection bactérienne consiste à incorporer dans le produit lui-même un conservateur chimique.

Mais ces conservateurs sont responsables d'une altération des qualités du produit, en particulier pour des produits pharmaceutiques et cosmétiques où ils génèrent, même parfois, des effets secondaires indésirables pour le consommateur.

La présente invention a pour but de résoudre les problèmes techniques précédents en combinant des moyens d'étanchéité mécaniques avec un agent antibactérien.

Ce but est atteint selon l'invention par un dispositif de pulvérisation d'un produit liquide contenu dans un récipient, comprenant notamment un embout destiné à coiffer le gicleur d'une pompe montée sur ledit récipient au moyen d'une collerette, ledit embout comportant un conduit interne prolongeant, d'une part, le tube gicleur jusqu'à une cavité d'extrémité pourvue d'un orifice d'éjection, dans laquelle est logé un clapet, et entouré coaxialement, d'autre part, d'une paroi externe cylindre-conique raccordée en partie basse à un épaulement d'appui pour la manoeuvre de la pompe caractérisé en ce que ledit conduit interne est réalisé avec une section annulaire et autour d'une tige axiale tandis que ladite cavité d'extrémité est fermée et est délimitée à l'intérieur de l'embout par sa paroi frontale percée de l'orifice d'éjection, d'une part, et par l'extrémité longitudinale aval de ladite tige axiale, d'autre part, et en ce que tous les éléments constitutifs susceptibles d'être en contact avec le produit et en particulier l'embout, sont réalisés, au moins partiellement, avec une matière plastique contenant entre 0,2% et 2% en poids d'un agent bactéricide qui n'agit que par contact et sans relarguage dans le produit.

Selon un mode de réalisation particulier, ledit agent bactéricide est un composé contenant de l'argent sous forme ionique.

Selon une caractéristique avantageuse, l'épaulement de l'embout se prolonge par un manchon cylindrique inférieur assurant, à la fois, le guidage axial de l'embout dans le col du récipient et la butée de fin de course sur la collerette de la pompe.

De préférence, ledit épaulement a un empattement supérieur au diamètre extérieur du col du récipient.

Selon une autre caractéristique, ledit clapet est amovible et constitué d'un corps cylindrique plein, pourvu à sa base d'une lèvre périphérique élastiquement déformable prenant appui sur les parois latérales internes de ladite cavité et orientée vers l'orifice d'éjection.

Avec le dispositif de l'invention, la protection antibactérienne n'est assurée que par des éléments mécaniques et sans que la composition chimique du produit ne soit modifiée.

Le clapet d'extrémité complète de manière efficace l'étanchéité du dispositif, en formant une barrière de protection supplémentaire qui vient s'ajouter aux clapets de la pompe.

L'action bactéricide est assurée par un agent chimique présent dans la matière plastique constitutive de l'embout. Cet agent n'est pas relargué dans le produit liquide et il n'agit que par contact avec ledit produit lorsque celui-ci passe dans l'embout, ou dans tout autre élément constitutif du dispositif, où cet agent est présent.

En présence d'une pollution bactérienne, les différents clapets du dispositif (embout, pompe) créent autant de niveaux d'étanchéité susceptibles de stopper la dissémination avant qu'elle n'atteigne le produit, tandis que l'agent bactéricide élimine complètement la pollution, là où elle est arrêtée.

Le moulage de l'embout avec une matière plastique contenant un agent bactéricide apporte une fonction hygiénique au dispositif. En effet, si l'embout est mis en contact avec une contamination bactérienne, il est pollué en surface. L'agent bactéricide présent va alors détruire les bactéricides polluantes et maintenir l'embout stérile, en prévenant toute nouvelle contamination.

La présente invention résulte donc de la combinaison entre des moyens mécaniques d'étanchéité et des moyens chimiques bactéricides.

L'invention sera mieux comprise à la lecture de la description qui va suivre accompagnée des dessins sur lesquels :
- la figure 1 représente une vue en coupe d'un mode de réalisation qui n'est pas couvert par les revendications, mais est conservé à titre d'explication.
- la figure 2 représente une vue en coupe d'une variante du mode de réalisation de la figure 1;
- les figures 3a et 3b représentent, respectivement, des vues, en coupe et de dessus, agrandies du mode de réalisation des figures 1 et 2;
- les figures 4a et 4b représentent, respectivement, des vues, en coupe et de dessus, d'un mode de réalisation de l'invention.

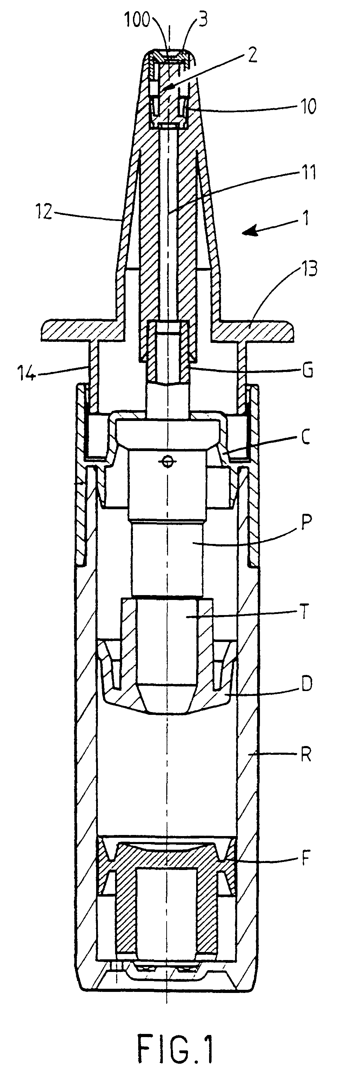

La figure 1 représente une vue en coupe d'un premier mode de réalisation d'un dispositif comprenant un récipient R contenant un produit liquide, une pompe P montée de façon étanche sur le récipient R au moyen d'une collerette C et équipée d'un tube gicleur G destiné à être coiffé par un embout 1. Le récipient R comporte, en outre, un fond mobile F tandis que le pied T de la pompe P est équipé d'une douille D coopérant avec le fond F, pour purger le récipient R.

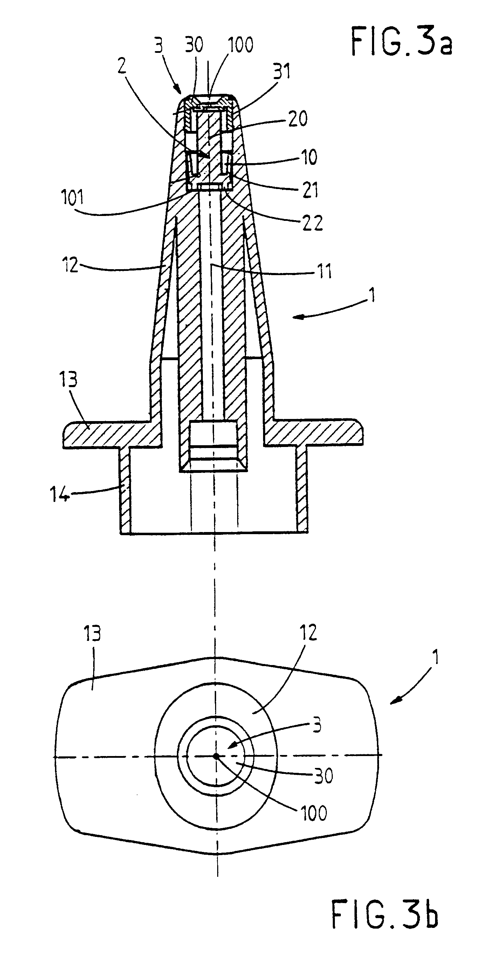

De manière générale, l'embout 1 comporte un conduit interne 11 prolongeant, le tube gicleur G, jusqu'à une cavité 10 d'extrémité, pourvue d'un orifice d'éjection 100 et dans laquelle est logé un clapet 2, comme représenté sur les figures 3a, 3b.

Le conduit interne 11 est entouré, coaxialement, d'une paroi externe cylindro-conique 12 raccordée en partie basse à un épaulement 13 s'étendant transversalement et destiné à l'appui manuel pour la manoeuvre de la pompe P.

L'épaulement 13 est pourvu d'un manchon cylindrique inférieur 14 assurant, à la fois, le guidage axial de l'embout 1 dans le col du récipient R et la butée de fin de course sur la collerette C de la pompe P.

L'épaulement 13 a un empattement qui est supérieur au diamètre extérieur du col du récipient R de façon à offrir une surface d'appui efficace.

L'embout 1 est réalisé, au moins, au niveau des parois internes du conduit 11, de la cavité 10 et du clapet 2, en contact avec le produit, en une matière plastique contenant un agent bactéricide.

Bien entendu, l'embout 1 peut être réalisé intégralement avec une matière plastique bactéricide.

Dans le cas où l'embout est moulé par injection ou compression, on peut prévoir que l'agent bactéricide soit mélangé avec la matière première avant le moulage.

Il est également possible de prévoir que tous les éléments constitutifs du dispositif (embout 1, corps, piston, et gicleur de la pompe P, récipient R, collerette C ...) sont réalisés avec une matière plastique bactéricide de façon à renforcer cette action.

L'agent bactéricide est de préférence un composé contenant de l'argent, et par exemple, de l'argent sous forme ionique.

L'agent bactéricide agit par contact avec le produit. C'est la raison pour laquelle, il est impératif que les zones de l'embout 1 et, le cas échéant, du dispositif de pulvérisation dans son ensemble, susceptibles d'être en contact avec le produit, contiennent une quantité suffisante d'agent bactéricide.

La quantité considérée comme efficace dans le domaine des produits considérés est comprise entre 0,2 % et 2% en poids d'agent antibactérien dans la matière plastique

A titre d'exemple, une telle matière permet de faire régresser de façon logarithmique et en 24 heures, une population bactérienne initiale du type staphyloccocus Aureus de 10⁵ à une population finale inférieure à 10².

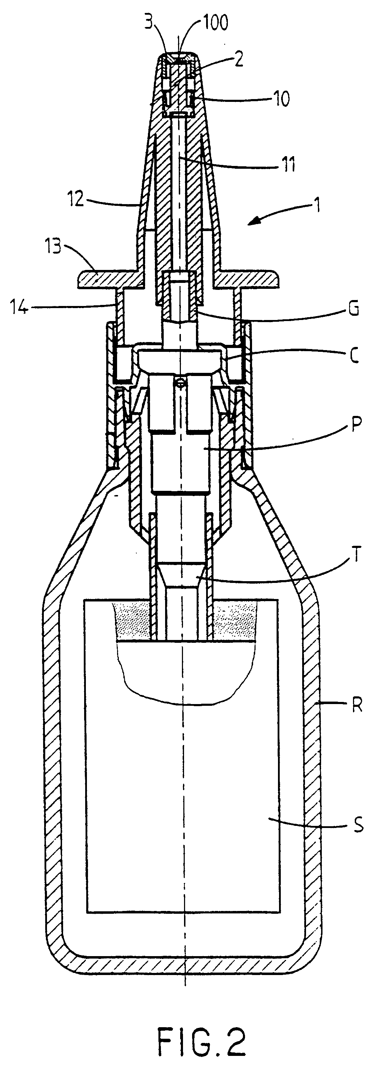

La figure 2 représente une vue en coupe d'une seconde variante de réalisation, dans laquelle le récipient R renferme une poche souple déformable S soudée au pied T de la pompe P.

Le mode de réalisation de l'embout 1 est ici identique à celui des figures 1 et 3a,3b.

Dans ce mode de réalisation, le conduit interne 11 est central et axial. Il débouche, à son extrémité supérieure, dans la cavité 10 qui reçoit le clapet 2 de façon amovible.

La cavité 10 est ici ouverte et délimitée à l'intérieur de l'embout 1 par le rebord annulaire 101 du conduit interne 11.

Le clapet 2 est constitué d'un corps cylindrique plein 20, pourvu à sa base d'une lèvre périphérique 21 élastiquement déformable qui prend appui sur les parois latérales internes de la cavité 10.

La lèvre périphérique 21 est orientée vers l'orifice d'éjection 100 situé au débouché de la cavité 10.

Par manoeuvre de la pompe P, le produit est mis sous pression et s'échappe par le tube gicleur G vers l'orifice d'éjection 100, via le conduit interne 11, en déformant dans la cavité 10, la lèvre périphérique 21 du clapet 2.

La lèvre 21 se déforme en se rabattant vers le corps cylindrique 20 du clapet 2 et en libérant ainsi un passage latéral pour le produit.

Le clapet 2 est retenu dans la cavité 10 au moyen d'une buse 3 qui obture, au moins partiellement, l'embouchure de la cavité 10.

La buse 3 comporte une face frontale 30 en cuvette percée d'un orifice central pour l'éjection du produit, et une jupe latérale 31, dont le bord inférieur est pourvu d'un organe d'encliquetage dans la cavité 10.

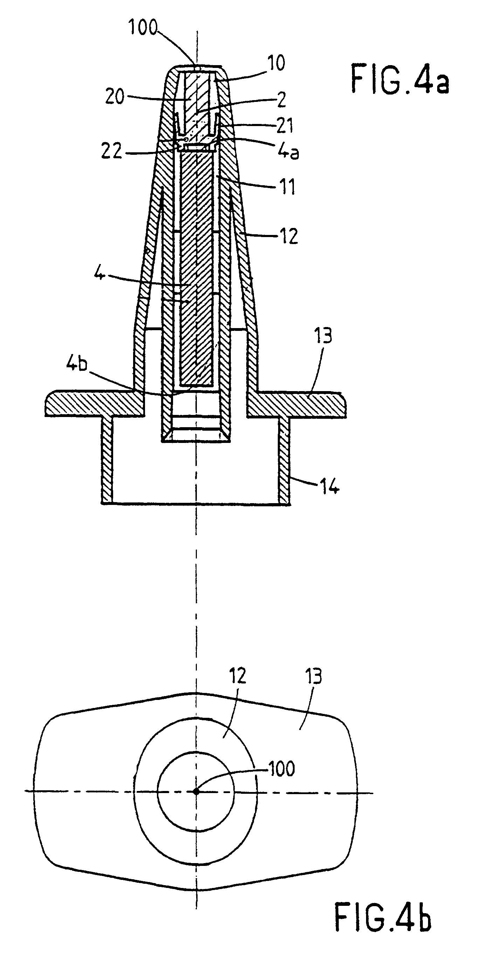

Conformément à l'invention, dans le mode de réalisation des figures 4a et 4b, le conduit interne 11 est réalisé avec une section annulaire et autour d'une tige axiale amovible 4. Dans ce cas, la cavité 10 est fermée et est délimitée, à l'intérieur de l'embout 1, par sa paroi frontale percée de l'orifice d'éjection 100, d'une part, et par l'extrémité longitudinale aval 4a de la tige axiale amovible 4, d'autre part.

Le clapet 2 est toujours amovible. Il est introduit dans la cavité 10 par le conduit 11 et est bloqué en force par la tige 4 contre la face interne de la paroi frontale de l'embout 1. Le cas échéant, la tige 4 est solidaire par son extrémité aval 4a du clapet 2. L'extrémité longitudinale amont 4b de la tige 4 est disposée, en position de montage du clapet, à distance du débouché du tube gicleur G, pour ne pas gêner l'échappement du produit.

## Revendications

1. Dispositif antibactérien de pulvérisation d'un produit liquide contenu dans un récipient (R), comprenant notamment un embout (1) destiné à coiffer le gicleur (G) d'une pompe (P) montée sur ledit récipient (R) au moyen d'une collerette (C) ; ledit embout (1) comporte un conduit interne (11) prolongeant, d'une part, le tube gicleur (G) jusqu'à une cavité (10) d'extrémité pourvue d'un orifice d'éjection (100), dans laquelle est logé un clapet (2), et entouré coaxialement, d'autre part, d'une paroi externe cylindro-conique (12) raccordée en partie basse à un épaulement (13) d'appui pour la manoeuvre de la pompe (P), **caractérisé en ce que** ledit conduit interne (11) est réalisé avec une section annulaire et autour d'une tige axiale (4) tandis que ladite cavité d'extrémité (10) est fermée et est délimitée à l'intérieur de l'embout (1) par sa paroi frontale percée de l'orifice d'éjection (100), d'une part, et par l'extrémité longitudinale aval (4a) de ladite tige axiale (4), d'autre part, et
**en ce que** tous les éléments constitutifs susceptibles d'être en contact avec le produit et en particulier l'embout (1), sont réalisés, au moins partiellement, avec une matière plastique contenant entre 0,2% et 2% en poids d'un agent bactéricide qui n'agit que par contact et sans relarguage dans le produit.

2. Dispositif selon la revendication 1, **caractérisé en ce que** ledit agent bactéricide est un composé contenant de l'argent sous forme ionique.

3. Dispositif selon l'une des revendications précédentes, **caractérisé en ce que** ledit épaulement (13) de l'embout (1) se prolonge par un manchon cylindrique inférieur (14) assurant, à la fois, le guidage axial de l'embout (1) dans le col du récipient (R) et la butée de fin de course sur la collerette (C) de la pompe (P).

4. Dispositif selon l'une des revendications précédentes, **caractérisé en ce que** ledit épaulement (13) de l'embout (1) a un empattement supérieur au diamètre extérieur du col du récipient (R).

5. Dispositif selon l'une des revendications précédentes, **caractérisé en ce que** ledit clapet (2) est amovible et constitué d'un corps cylindrique plein (20) pourvu à sa base d'une lèvre périphérique (21) élastiquement déformable, prenant appui sur les parois latérales internes de ladite cavité (10) et orientée vers l'orifice d'éjection (100).

## Claims

1. An antibacterial spray device for spraying a liquid contained in a receptacle (R), the device comprising, in particular, an endpiece designed to cover the outlet tube (G) of a pump (P) which is mounted on said receptacle (R) by means of a collar (C); said endpiece (1) includes an internal duct (11) which firstly extends from the outlet tube (G) to an end cavity (10) which is provided with an ejection orifice (100), and in which a valve (2) is housed, and secondly, is surrounded coaxially by a tapering cylindrical outside wall (12) which is attached at its bottom portion to a bearing shoulder (13) for operating the pump (P), the device being **characterized in that** said internal duct (11) is of annular section, about an axial rod (4), while said end cavity (10) is closed and is defined inside the endpiece (1) firstly by its front wall which is pierced by the ejection orifice (100), and secondly by the downstream longitudinal end (4a) of said axial rod (4), and
**in that** all the component parts that can come into contact with the liquid, and in particular the endpiece (1), are made, at least in part, of a plastics material containing between 0.2% and 2% by weight of a bactericidal agent which acts solely by coming into contact with the liquid but without being released into it.

2. A device according to claim 1, **characterized in that** said bactericidal agent is a compound containing silver in ionic form.

3. A device according to any preceding claim, **characterized in that** said shoulder (13) of the endpiece (1) is extended by a bottom cylindrical sleeve (14) which ensures that the endpiece (1) is axially guided inside the neck of the receptacle (R) and comes into abutment with the collar (C) of the pump (P).

4. A device according to any preceding claim, **characterized in that** said shoulder (13) of the endpiece (1) has a span which is greater than the outside diameter of the neck of the receptacle (R).

5. A device according to any preceding claim, **characterized in that** said valve (2) is removable and constituted by a solid cylindrical body (20) provided at its base with an elastically-deformable peripheral lip (21) which bears against the internal side walls of said cavity (10) and is oriented towards the ejection orifice (100).

## Patentansprüche

1. Antibakterielle Sprühvorrichtung für eine Flüssigkeit, die in einem Behälter (R) enthalten ist, insbesondere umfassend einen Aufsatz (1), der zum Bedecken der Spritzdüse (G) einer Pumpe (P) bestimmt ist, die mittels eines Flansches (C) am Behälter (R) angebracht ist; dieser Aufsatz (1) umfasst eine innere Leitung (11), die auf der einen Seite die Düsenröhre (G) bis zu einer Endausnehmung (10) verlängert, welche mit einer Ausstoßöffnung (100) versehehen ist, in der eine Klappe (2) angeordnet ist, und auf der anderen Seite von einer zylindrisch-konischen Außenwand (12) koaxial eingefasst ist, die im unteren Teil für die Bedienung der Pumpe (P) mit einem Stützvorsprung (13) verbunden ist, **dadurch gekennzeichnet, dass** die innere Leitung (11) mit einem Abschnitt ausgeführt ist, der ringförmig um einen Axialschaft (4) herumgeht, wohingegen die Endausnehmung (10) verschlossen und innerhalb des Aufsatzes (1) auf der einen Seite von ihrer von der Ausstoßöffnung (100) durchbrochenen Stirnwand und auf der anderen Seite vom stromabwärtigen Längsendteil (4a) des Axialschafts (4) begrenzt ist, und dadurch,
dass alle wesentlichen Elemente, die dazu geeignet sind, mit dem Produkt und insbesondere dem Aufsatz (1) in Kontakt zu sein, zumindest teilweise aus einem Kunststoffmaterial ausgeführt sind, welches zwischen 0,2 % und 2 % Gewichtsprozent eines nur durch Kontakt und ohne Aussalzung in dem Produkt wirkenden bakteriziden Mittels enthält.

2. Behälter nach Anspruch 1, **dadurch gekennzeichnet, dass** das bakterizide Mittel eine Silber in ionischer Form enthaltende Verbindung ist.

3. Behälter nach einem der vorhergehenden Ansprüche, **dadurch gekennzeichnet, dass** sich der Vorsprung (13) des Aufsatzes (1) durch eine untere zylindrische Muffe (14) fortsetzt, welche zugleich die axiale Führung des Aufsatzes (1) im Hals des Behälters (R) und den Endanschlag am Flansch (C) der Pumpe (P) sicherstellt.

4. Behälter nach einem der vorhergehenden Ansprüche, **dadurch gekennzeichnet, dass** der Vorsprung (13) des Aufsatzes (1) eine Spanne hat, die größer als der Außendurchmesser des Halses des Behälters (R) ist.

5. Behälter nach einem der vorhergehenden Ansprüche, **dadurch gekennzeichnet, dass** die Klappe (2) abnehmbar ist und aus einem vollen zylindrischen Körper (20) gebildet ist, der in seinem Unterteil mit einer elastisch verformbaren peripheren Lippe (21) ausgestattet ist, die an den inneren Seitenwänden der Ausnehmung (10) anliegt und zur Ausstoßöffnung (100) gerichtet ist.
